# EUROPÄISCHE PATENTANMELDUNG

(11) **EP 0 579 343 A2**
(43) Veröffentlichungstag der Anmeldung: **19.01.1994**
(21) Anmeldenummer: 93250140.6
(22) Anmeldetag: 13.05.1993
(51) Int. Cl.: G01N 33/543, G01N 33/80

(54) **Verfahren und Vorrichtung zum Nachweis von Analyten in Flüssigkeiten**

(30) Priorität: 15.07.1992 DE 4223791
(71) Anmelder: EPPENDORF-NETHELER-HINZ GMBH GEWERBLICHER RECHTSSCHUTZ, W-2000 Hamburg 63 (DE)
(72) Erfinder: Hofmeier, Gerhard, Dr., W-2000 Hamburg 65 (DE); Goemann, Wolfgang, Dr., W-2000 Hamburg 67 (DE); Lurz, Werner, Dr., W-2358 Kaltenkirchen (DE); Scheller, Frieder, Prof.Dr., O-1297 Zepernick (DE)
(74) Vertreter: UEXKÜLL & STOLBERG

(57) **Zusammenfassung**

Die Erfindung betrifft ein Verfahren zum Nachweis von Analyten (und partikulären Analyten) in Flüssigkeiten durch optische oder elektrochemische Verfahren. Die nachzuweisenden Analyte reagieren mit einem den Analyten spezifisch bindenden Angenz, das entweder auf einer Sensoroberfläche immobilisiert ist oder sich in der zu untersuchenden Flüssigkeit befindet. In beiden Fällen führt die Reaktion zu einer Sperrwirkung, so daß die Zugänglichkeit der Sensoroberfläche für signalvermittelnde Stoffe eingeschränkt wird.

## Beschreibung

Die vorliegende Erfindung betrifft ein Verfahren zum Nachweis von Analyten und partikulären Analyten in Flüssigkeiten gemäß dem Oberbegriff des Anspruchs 1 sowie eine Vorrichtung gemäß dem Oberbegriff des Anspruchs 24.

Die WO 89/08713 (PCT/US 89/01057) beschreibt amperometrische Analyseverfahren und Vorrichtungen zu Bestimmung der Konzentration von Glukose, Cholesterin und anderen Analyten im Blut. Die Vorrichtungen bestehen aus einmal zu verwendenden Sensoren und einem wieder verwendbaren, tragbaren Meßgerät. Die nachzuweisenden Analyte, Glukose bzw. Cholesterin, werden durch ein zugesetztes Oxidationsmittel enzymatisch oxidiert. Anschließend wird das reduzierte Oxidationsmittel elektrochemisch reoxidiert, wobei eine definierte Spannung angelegt und die resultierende diffusionskontrollierte Stromstärke gemessen wird. Die Spezifität dieses Verfahrens beruht auf der Spezifität der enzymatischen Reaktion und ist daher auf solche Stoffe begrenzt, die sich enzymatisch oxidieren lassen.

Die WO 84/04391 (PCT/US 84/00374) beschreibt Verfahren zur Bestimmung von Aggregaten partikulärer Stoffe, wie sie z.B. auch bei der Immunreaktion entstehen können. Die Messungen werden an individuellen Aggregaten, die zu einer bestimmten Klasse gehören, vorgenommen. Hierzu wird ein repräsentatives Feld der Suspension in dicht benachbarte Meßpunkte aufgeteilt. Die zu messende Größe wird für jeden Meßpunkt getrennt bestimmt, gespeichert und mit Hilfe eines Computers ausgewertet. Auf diese Weise wird die Zahl von Aggregaten, die einer bestimmten Aggregatklasse angehören, ermittelt. Diese Zahl erlaubt eine Aussage über das Ausmaß der Agglutination und die Quantifizierung des Analytes. Dieses Verfahren erfordert eine genaue Auswahl geeigneter Aggregatklassen sowie ein geeignetes Computerprogramm. Bei dem in der WO 84/04391 beschriebenem Meßverfahren handelt es sich hauptsächlich um eine optisches Verfahren.

Aufgabe der Erfindung ist die Schaffung eines einfachen immunochemischen Verfahrens, mit dem Analyte und partikuläre Analyte in einer Flüssigkeit nachgewiesen und bestimmt werden können.

Gelöst wird diese Aufgabe für ein Verfahren durch die Merkmale im Anspruch 1 und für eine Vorrichtung durch die Merkmale im Anspruch 24.

Erfindungsgemäß zeigte sich, daß solche spezifischen quantitativen Bestimmungen unter Ausnutzung des Ligandentests durchgeführt werden können, wenn die spezifische Bindungsreaktion in Anwesenheit einer oder mehrerer signalvermittelnder Substanzen auf bzw. benachbart zu einer Sensoroberfläche durchgeführt werden. Hierbei wird die Zugänglichkeit der Sensoroberfläche für die signalvermittelnden Substanzen als Folge der spezifischen Bindungsreaktion eingeschränkt.

Unter Ligandentest wird ein Test verstanden, der für die qualitative oder quantitative Messung eines Analyten die spezifische Bindung des Analyten an ein Reagenz ausnutzt, beispielsweise Antigen-Antikörper-Bindung, Hormon-Rezeptor-Bindung, Biotion-Streptavidin-Bindung oder Kohlehydrat-Lektin-Bindung.

Zum Nachweis von Analyten in Flüssigkeiten werden Sensoroberflächen verwendet, die mit einer porösen Schicht überzogen sind. Als signalvermittelnde Substanzen finden partikuläre Agenzien Anwendung, deren Durchmesser nicht größer als die Poren der porösen Schicht der Sensoroberfläche sind, so daß die monomeren Partikel nahezu ungehindert an die Sensoroberfläch gelangen können, wo sie aufgrund ihrer signalvermittelnden Eigenschaften zu einem Meßeffekt führen. Die partikulären Agenzien besitzen neben den signalvermittelnden auch für den nachzuweisenden Analyten spezifische, bindende Eigenschaften.

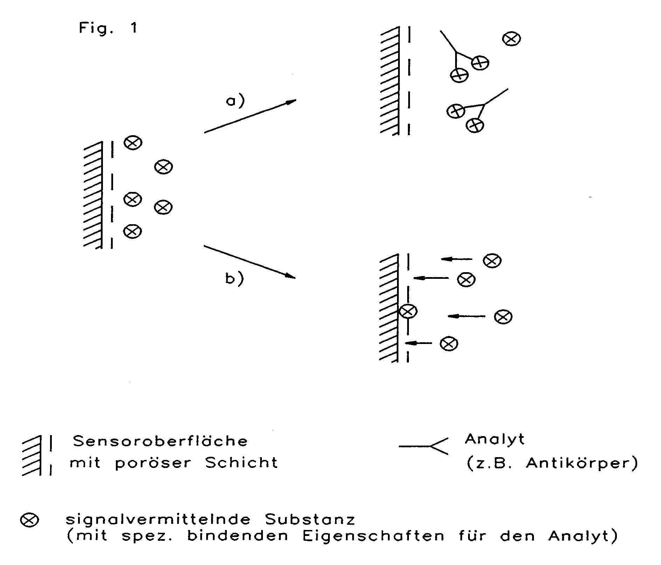

Wird die zu untersuchende Flüssigkeit in Gegenwart der signalvermittelnden Substanz mit der Sensoroberfläche in Kontakt gebracht, reagiert der nachzuweisende Analyt spezifisch mit dem partikulären signalvermittelnden Stoff. Die Reaktionsbedingungen sind so zu wählen, daß ein Teilchen des Analyten mit mindestens zwei Teilchen des partikulären Agens reagiert, wobei ein Komplex aus mindestens zwei solcher Teilchen gebildet wird. Der so gebildete Komplex ist aufgrund seiner Größe nicht mehr in der Lage die Poren der besagten porösen Schicht zu passieren, und gelangt also nicht auf die Sensoroberfläche (Figur 1, Weg a).

Befindet sich der nachzuweisende Analyt nicht in der Flüssigkeit, findet keine Agglutination zwischen dem partikulären signalvermittelnden Stoff statt, so daß dieser ungehindert durch die poröse Membran auf die Sensoroberfläche gelangen kann (Figur 1, Weg b).

Zum Nachweis partikulärer Analyte werden Sensoroberflächen eingesetzt, auf denen den Analyten spezifisch bindende Agenzien fixiert sind. Alternativ kann das bindende Agens an eine die Sensoroberfläche überziehenden porösen Schicht immobilisiert werden. Die Porengröße dieser Schicht darf dabei nicht kleiner sein als die Größe der Teilchen (Ionen, Moleküle, Komplexe), die die signalvermittelnde Substanz darstellen.

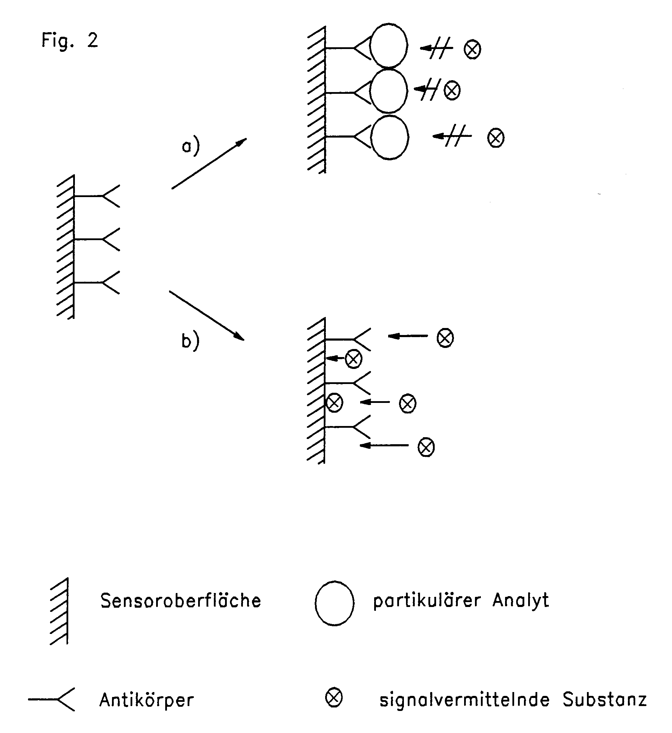

Wird die Sensoroberfläche mit der zu untersuchenden Flüssigkeit in Kontakt gebracht, wird der nachzuweisende partikuläre Analyt von den auf der Sensoroberfläche angebrachten Liganden gebunden und bildet dort eine Schicht, die die freie Zugänglichkeit der Sensoroberfläche für die signalvermittelnde Substanz vermindert (Figur 2, Weg a).

Enthält die zu untersuchende Flüssigkeit den nachzuweisenden Analyten nicht, wird die Zugänglichkeit der Sensoroberfläche für die signalvermittelnde Substanz nicht beeinträchtigt (Figur 2, Weg b).

Zur Verhinderung unspezifischer Reaktionen kann die Sensoroberfläche nach der Fixierung des bindenden Agens derart behandelt werden, daß eine unspezifische Bindung von Substanzen an die Sensoroberfläche verhindert wird. Bei Antigen-Antikörper-Bindungen kann beispielsweise die Sensoroberfläche zunächst mit Antikörper als dem bindenden Agens belegt und anschließend mit Albumin zur Blockierung unspezifisch bindungsaktiver Stellen behandelt werden.

Die hierbei verwendeten signalvermittelnden Substanzen zeigen keine spezifisch bindenden Eigenschaften und brauchen nicht partikulärer Natur zu sein, jedoch läßt sich die Sperrwirkung der Schicht der gebundenen partikulären Analyte durch Verwendung voluminöser signalvermittelnder Substanzen vergrößern. Hierzu können die signalvermittelnden Substanzen z.B. an Latexpartikel gebunden werden.

Als bindende Agenzien eignen sich alle Substanzen, die zu einer spezifischen Bindung in der Lage sind; besonders geignet sind Lectine, Antikörper, Hormon-Rezeptoren, Avidin oder Streptavidin. Da spezifisch bindende Antikörper für nahezu jeden belieben Stoff über Standardverfahren erhalten werden können, kommt diesen bei dem erfindungsgemäßen Verfahren eine besondere Bedeutung zu.

Die Porengröße der porösen Schichten auf der Sensoroberfläche wird entsprechend der Größe der als signalvermittelnde Substanzen eingesetzten partikulären Agenzien gewählt. Geeignet sind z.B. Kapillar-Poren-Membranen (Nuclepore®), die mit genau definierten Porengrößen bis ca. 20 µm erhältlich sind, oder auch mikroporöse Membranen auf Zellulosebasis.

Als Detektionssysteme können sowohl elektrochemische als auch optische Sensoren eingesetzt werden.

In Fall der elektrochemischen Verfahren werden Sensoroberflächen aus einem elektrisch leitenden Material und als signalvermittelnde Substanzen elektroaktive Materialien, vorzugsweise Redoxsysteme eingesetzt.

Als Material für die Sensoroberfläche eignen sich alle elektrisch leitenden Stoffe, die sowohl metallischer als auch nichtmetallischer Natur sein können. Bevorzugtes Material für die Sensoroberfläche ist Glaskohle. Die Sensoroberfläche kann auch als dünne Schicht auf einem planaren Substrat aufgebracht sein.

Als signalvermittelnde Substanzen kommen vorzugsweise lösliche Redoxsysteme in Betracht; besonders geeignet sind Ferrocen und seine Derivate, Hexacyanoferrat, Benzochinon oder Tetramethyl-p-phenylendiamin.

Die partikulären signalvermittelnden Substanzen werden durch die Fixierung von elektroaktiven Materialien auf Partikeln, beispielsweise Latexpartikeln, gebildet. Besonders geeignet ist hier ebenfalls Ferrocen.

Die Bindung des nachzuweisenden Analyten an das partikuläre signalvermittelnde Agens bzw. an die Sensoroberfläche äußert sich in einer veränderten Stromspannungscharakteristik, die sich mit einer elektrochemischen Meßanordnung erfassen läßt. Hierzu eignen sich besonders Verfahren der Amperometrie oder der zyklischen Voltametrie.

Für die optischen Nachweisverfahren werden Sensoroberflächen aus optisch transparentem Material und als signalvermittelnde Substanzen lichtemittierende und/oder lichtabsorbierende Substanzen verwendet. Besonders geeignet sind Fluorochrome, beispielsweise Fluoreszein- oder Rhodamin-Derivate, Luminogene, beispielsweise Dioxetanderivate, oder Chromophore, beispielsweise Bromphenolblau oder Chlorphenolrot.

Partikuläre, signalvermittelnde Substanzen für die optischen Verfahren werden durch die Fixierung von lichtemittierenden und/oder lichtabsorbierenden Substanzen auf Partikeln, beispielsweise Latexpartikeln gebildet.

Die Bewegung von partikulären Agenzien zur Sensoroberfläche läßt sich dadurch beschleunigen, daß partikuläre Agenzien mit magnetischen Eigenschaften verwendet werden und gleichzeitig ein äußeres Magnetfeld angelegt wird. Derartige partikuläre Agenzien erhält man beispielsweise durch Überzug von magnetischen Partikeln, beispielsweise aus Ferrit, mit einem Polymer, beispielsweise Zellulose oder Latex.

Die Bewegung der partikulären Agenzien zur Sensoroberfläche läßt sich weiterhin durch Schwerkraft, z.B. durch Zentrifugation, oder durch Ausnutzung von Kapillarkräften, z.B. durch Verwendung poröser Sensoroberflächen, beschleunigen.

Die partikulären Agenzien können sowohl biologischer Herkunft, bevozugt sind Zellen, oder künstlich hergestellt sein, bevorzugt sind Latexpartikel.

Das beschriebene Verfahren eignet sich zum Nachweis beliebiger Analyte. Besonders geeignet ist es zum Nachweis blutgruppenspezifischer Antigene auf Erythrozyten, Antikörpern gegen diese blutgruppenspezifischen Antigene und definierter Zelltypen mittels spezifischer Antigene oder Kohlehydratstrukturen auf der Zellmembran.

Die Sensoroberfläche kann auf einem zur einmaligen Verwendung gedachten Teil zusammengefaßt werden, das zusätzlich auch die signalvermittelnde Substanz enthalten kann. Das einmal zu verwendende Teil wird in Kombination mit einem mehrfach zu verwendenden Meßgerät eingesetzt.

Im folgenden wird die Erfindung anhand von Beispielen erläutert.

### Beispiel 1

Physikalische Adsorption von Antikörpern

Zum Nachweis partikulärer Analyte wird von einem Glaskohlerundstab (Fa. SIGRI GmbH, Meitingen, Deutschland) mit einem Durchmesser von 6 mm eine etwa 5 mm dicke Scheibe abgeschnitten. Eine Seite der Scheibe wird poliert, die andere Seite wird mit einem Draht elektrisch leitend verbunden.

Die polierte Seite der Glaskohlenscheibe wird in eine Pufferlösung (pH 9,5) getaucht, die etwa 1 mg/ml Anti-A- bzw. Anti-B-Antikörper enthält (monoklonale Antikörper des Typs IGM, gereinigt durch Affinitätschromatigraphie, Fa. Chembiomed, Edmonton, Alberta, Kanada). Nach Inkubation für etwa eine Stunde wird die polierte Oberfläche sorgfältig mit Pufferlösung abgespült und für eine weitere Stunde in einer Albuminlösung inkubiert (10 mg Albumin/ml). Nach der zweiten Inkubation wird erneut mit Pufferlösung gespült.

### Beispiel 2

Chemische Immobilisierung von Antikörpern

Die chemische Immobilisierung von Antikörpern an die Glaskohleoberfläche erfolgt in an sich bekannter Weise (G. A. Robinson et al., Biosensors **2** (1986) 45). Wie in Beispiel 1 beschrieben, wird von einem Glaskohlerundstab (Durchmesser 6 mm) eine etwa 5 mm dicke Scheibe abgeschnitten und eine Seite der Scheibe poliert, die andere Seite wird mit einem Draht elektrisch leitend verbunden. Die polierte Seite der Glaskohlescheibe wird durch Eintauchen in 10%iger Salpetersäure, die 2,5% K₂Cr₂O₇ enthält, und Anlegen einer Spannung von + 2,2 Volt, geschaltet gegen eine Standard-Kalomel-Elektrode, 10 Sekunden oxidiert. Anschließend wird die Sensoroberfläche mit Pufferlösung gespült und bei Raumtemperatur für 80 Minuten in einer 0,15 M 1-Cyclohexyl-3-(2-morpholinoethyl)-carbodiimid-p-methyltoluol-sulfonat-Lösung (Sigma-Chemie, Deisenhofen, Deutschland) in 0,1 M Acetatpuffer (pH 4,5) inkubiert. Nach erneutem Spülen mit Pufferlösung wird die aktivierte Sensoroberfläche weiterbehandelt, wie in Beispiel 1 beschrieben.

### Beispiel 3

Messung

Zur Bestimmung des nachzuweisenden Analyten wird eine Durchflußmeßzelle verwendet, welche die mit Antikörpern beladene Sensoroberfläche und eine Gegenelektrode enthält; als Referenzelektrode dient eine Kalomel-Elektrode. Die Strömungsgeschwindigkeit des zu untersuchenden Mediums wird auf etwa 2,2 ml/min eingestellt und mittels einer Potentiostatenschaltung der Sensor auf ein Potential von + 400 mV gegen die Referenzelektrode polarisiert. Mit einem Strom-Spannungswandler wird der über die Sensoroberfläche fließende Strom gemessen und mit einem x-t-Schreiber registriert.

Die eigentliche Messung erfolgt in sechs Schritten:
(i) Festlegen der Nullinie durch Spülen der Meßzelle für etwa 60 Sekunden mit eine 0,15 M NaCl-Lösung in Puffer (pH 7,5).
(ii) Bestimmung des Referenzsignals durch Leiten einer etwa 2 mM-Ferrocenlösung in Puffer (pH 7,5, 0,15 M NaCl) durch die Meßzelle. Es wird solange mit Ferrocenlösung gespült, bis die Stromstärke einen konstanten Wert erreicht, etwa 2 Minuten. Hierbei wird ein deutlicher Anstieg des über die Sensoroberfläche fließenden Stroms registriert (etwa 30 µA).
(iii) Spülen der Meßzelle mit Puffer bis zum Erreichen der Nullinie.
(iv) Leiten von Vollblut der Blutgruppe A durch die Meßzelle (bis zum Erreichen einer konstanten Stromstärke, etwa 2 Minuten).
(v) Spülen der Meßzelle mit Puffer bis zum Erreichen der Nullinie.
(vi) Erneutes Leiten einer 2 mM Ferrocenlösung durch die Meßzelle und Registrieren der Stromstärke.

### Auswertung:

Bei Verwendung des mit Antikörpern gegen die Blutgruppe A beschichteten Sensors liegt die registrierte Stromstärke (etwa 25 µA) deutlich unter dem Referenzsignal, bei Verwendung des mit Antikörpern gegen die Blutgruppe B beschichteten Sensors erreicht die Stromstärke die Höhe des Referenzsignals.

**Figur** **1** zeigt schematisch den Nachweis von Analyten. Die mit einer porösen Schicht überzogene Sensoroberfläche wird in Gegenwart der signalvermittelnden Substanz mit der zu untersuchenden Flüssigkeit in Kontakt gebracht. Enthält diese den nachzuweisenden Analyten, reagiert er spezifisch mit dem partikulären signalvermittelnden Stoff (Weg a). Die dabei gebildeten Komplexe können aufgrund ihrer Größe die Poren der porösen Schictht nicht mehr passieren.

Enthält die zu untersuchende Flüssigkeit den nachzuweisenden Analyten nicht, gelangt der partikuläre Stoff ungehindert zur Sensoroberfläche (Weg b).

**Figur** **2** zeigt schematisch den Nachweis partikulärer Analyte. Die mit einem den Analyten spezifisch bindenden Agenz, z.B. Antikörpern, besetzte Sensoroberfläche wird mit der zu untersuchenden Flüssigkeit in Kontakt gebracht. Enthält diese den nachzuweisenden Analyten, wird er von dem bindenden Agenz an die Sensoroberfläche gebunden, wo er eine Schicht bildet, die die freie Zugänglichkeit der Sensoroberfläche für die signalvermittelnde Substanz vermindert (Weg a).

Enthält die zu untersuchende Flüssigkeit den nachzuweisenden Analyten nicht, gelangt der partikuläre Stoff ungehindert zur Sensoroberfläche (Weg b).

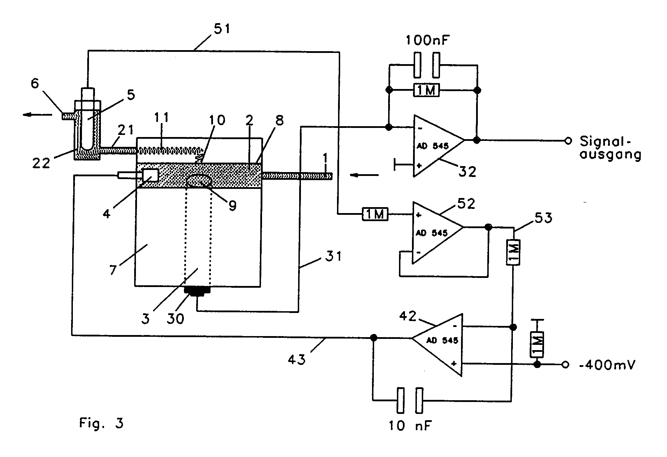

**Figur 3** zeigt eine Schaltung zur Durchführung des Verfahrens mit einem Kunststoffkörper 7, in den eine Meßkammer 8 eingeformt ist. Die Meßkammer 8 wird von einer Bohrung gebildet, an deren eines Ende ein Zulauf 1 ansetzt. Das dem Zulauf 1 gegenüberliegende andere Ende ist durch den Kopf 4 einer Hilfselektrode 2 verschlossen. In der Zeichnung ist dies nur schematisch dargestellt. Die Hilfselektrode 2 ist ein zylindrisches Gitter aus elektrisch leitendem Material, das in die Meßkammer 8 eingesetzt ist. In ihrem Mantel ist eine erste Öffnung 9 vorgesehen, durch die ein Kohlesensor 3 in die Meßkammer 8 gesteckt werden kann.

Die Hilfselektrode 2 weist eine weitere Öffnung 10 auf, an die ein Ablaufkanal 11 anschließt. In einer Ausführungsform sind die Öffnungen 9 und 10 unter 90° zueinander angeordnet. Die Öffnung 9 der Hilfelektrode 2 fluchtet mit einer Bohrung, in die der Kohlesensor 3 durch den Kunststoffkörper 7 gesteckt wird. Das freie Ende 30 des Kohlesensors 3 ragt aus dem Kunststoffkörper 7 heraus und dient zum Anschließen einer Signalleitung 31. Die Signalleitung 31 führt somit von dem Kohlesensor 3 zu dem negativen Eingang eines ersten Verstärkers 32, dessen positiver Eingang auf Masse liegt. Der Signalausgang des ersten Verstärkers 32 ist über einen Widerstand von mindestens 1 MΩ an den negativen Eingang rückgekoppelt. Parallel zu dem Widerstand liegt ein Kondensator von etwa 100 nF. Auf diese Weise werden die vom Kohlesensor aufgenommenen Stromsignale in Spannungssignale von wenigen Millivolt bis zu etwa 1 Volt umgewandelt und erscheinen für die weitere Verarbeitung am Signalausgang.

Der Ablaufkanal 11 ist über eine Leitung 21 mit dem Gehäuse 22 einer Referenzelektrode 5 verbunden. Das Gehäuse 22 weist einen Ablauf 6 auf. Die zu untersuchende Flüssigkeit strömt somit vom Zulauf 1 durch die Meßkammer 8 am Kopf des Kohlesensors 3 vorbei, durch die Öffnung 10 der Hilfselektrode 2 in den Ablaufkanal 11 und von diesem an der Referenzelektrode 5 vorbei in den Ablauf 6. Die Referenzelektrode 5 ist in einer Ausführungsform eine Kalomelelektrode, deren eines Ende über eine Leitung 51 mit dem positiven Eingang eines zweiten Verstärkers 52 verbunden ist. In der Leitung 51 liegt außerdem ein serieller Vorwiderstand von 1 MΩ. Der Ausgang des zweiten Verstärkers 52 ist auf seinen negativen Eingang rückgekoppelt. In der Ausgangsleitung 53 des zweiten Verstärkers 52 liegt ein weiterer Widerstand von 1 MΩ, der mit seinem anderen Anschluß am negativen Eingang eines dritten Verstärkers 42 liegt. Außerdem ist er mit einer Platte eines Kondensators von 10 nF verbunden, dessen andere Platte an den Ausgang des dritten Verstärkers 42 angeschlossen ist. Am positiven Eingang des dritten Verstärkers 42 liegen -400 mV. Die Ausgangsleitung 43 des dritten Verstärkers 42 ist an den Kopf 4 der Hilfselektrode 2 angeschlossen.

Die Referenzelektrode 5 soll immer auf 0 Volt liegen. Die Hilfselektrode 2 soll immer auf -400 mV liegen. Im wesentlichen bewirkt die Schaltung nun, daß zwischen der Hilfselektrode 2 und der Referenzelektrode 5 eine Potentialdifferenz von -400 mV aufrechterhalten bleibt. Verändert sich das Potential der Referenzelektrode 5, dann beeinflußt dies über den dritten Verstärker 42 die über die Leitung 43 an die Hilfselektrode 2 gelegte Spannung in der Weise, daß immer eine Potentialdifferenz von -400 mV aufrechterhalten wird.

Es wird darauf hingewiesen, daß in den Kunststoffkörper 7 so viele Kohlesensoren 3 eingesetzt sein können, wie beispielsweise Blutgruppen nachgewiesen werden sollen. Auf diese Weise kann der Nachweis für mehrere Blutgruppen gleichzeitig erfolgen. Es ist klar, daß dann für jeden weiteren Kohlesensor ein weiterer Verstärker vorgesehen sein muß. Die Verstärker sind im übrigen alle vom Typ AD 545.

## Patentansprüche

1. Verfahren zum Nachweis von Analyten in Flüssigkeiten nach dem Prinzip des Ligandentests, **dadurch gekennzeichnet,** daß eine mit einer porösen Schicht überzogene Sensoroberfläche mit einer Flüssigkeit in Kontakt gebracht wird, die neben dem nachzuweisenden Analyten ein oder mehrere partikuläre Agenzien enthält, die sowohl signalvermittelnde als auch für den Analyten spezifisch bindende Eigenschaften aufweisen,
- daß die Poren der porösen Schicht nicht kleiner als der Durchmesser der Partikel der partikulären Agenzien gewählt werden und den Durchtritt monomerer Partikel der partikulären Agenzien an die Sensoroberfläche gestatten;
- daß der nachzuweisende Analyt mit mindestens zwei Partikeln der partikulären Agenzien eine spezifische Bindung eingeht, wobei ein Komplex aus mindestens zwei Partikeln der partikulären Agenzien entsteht, der aufgrund seiner Größe die Poren der besagten porösen Schicht nicht mehr passieren kann; und
- daß der Sensor die Menge der auf seiner Oberfläche gelangten Partikel der partikulären Agenzien aufgrund der signalvermittelnden Eigenschaften feststellt.

2. Verfahren zum Nachweis partikulärer Analyte in Flüssigkeiten nach dem Prinzip des Ligandentests, **dadurch gekennzeichnet,** daß der nachzuweisende partikuläre Analyt an ein den Analyten spezifisch bindendes Agens gebunden wird, das auf einer Sensoroberfläche oder benachbart zu einer Sensoroberfläche immobilisiert ist, und daß die Flüssigkeit eine oder mehrere signalvermittelnde Substanzen enthält, wobei die Zugänglichkeit der Sensoroberfläche für die signalvermittelnde(n) Substanz(en) durch die von dem fixierten Agens gebundenen Partikel des partikulären Analyten eingeschränkt wird, so daß sich die direkte Umgebung der besagten Sensoroberfläche in einer durch den Sensor meßtechnisch erfaßbaren Weise ändert.

3. Verfahren nach Anspruch 1, **dadurch gekennzeichnet,** daß als Sensoroberfläche ein elektrisch leitendes Material verwendet wird und daß als signalvermittelnde partikuläre Agenzien mit Redoxsystemen gekoppelte Partikel verwendet werden.

4. Verfahren gemäß Anspruch 2, **dadurch gekennzeichnet,** daß als Sensoroberfläche ein elektrisch leitendes Material verwendet wird und daß als signalvermittelnde Substanzen Redoxsysteme verwendet werden und daß sich die Anderung der direkten Umgebung der Sensoroberfläche in einer veränderten Strom-Spannungscharakteristik äußert.

5. Verfahren nach Anspruch 3 oder 4, **dadurch gekennzeichnet,** daß als Sensoroberfläche Glaskohle verwendet wird.

6. Verfahren nach einem der Ansprüche 3 bis 5, **dadurch gekennzeichnet,** daß als signalvermittelndes partikuläres Agens auf Partikel fixiertes Ferrocen oder als signalvermittelnde Substanz Ferrocen verwendet wird.

7. Verfahren nach Anspruch 1 oder 2, **dadurch gekennzeichnet,** daß die Sensoroberfläche aus einem optisch transparenten Material hergestellt wird und daß als signalvermittelnde partikuläre Agenzien oder als signalvermittelnde Substanzen lichtemittierende und/oder lichtabsorbierende Stoffe verwendet werden.

8. Verfahren nach Anspruch 7, **dadurch gekennzeichnet,** daß als signalvermittelnde partikuläre Agenzien oder als signalvermittelnde Substanzen Fluorochrome oder Luminogene oder Chromophore verwendet werden.

9. Verfahren gemäß einem der Ansprüche 2 bis 8, **dadurch gekennzeichnet,** daß das Volumen der signalvermittelnden Substanzen durch Bindung an partikulärer Stoffe vergrößert und so die Sperrwirkung verstärkt wird.

10. Verfahren gemäß Anspruch 9, **dadurch gekennzeichnet,** daß als partikuläre Stoffe Latexpartikel verwendet werden.

11. Verfahren gemäß einem der Ansprüche 2 bis 10, **dadurch gekennzeichnet,** daß die Sensoroberfläche nach Immobilisierung des bindenden Agens derart behandelt wird, daß eine unspezifische Bindung des Analyten an die Sensoroberfläche verhindert wird.

12. Verfahren nach einem der Ansprüche 1 bis 11, **dadurch gekennzeichnet,** daß als nachzuweisender Analyt oder als an die Sensoroberfläche fixiertes Agens ein Antikörper oder ein Antigen verwendet wird.

13. Verfahren nach Anspruch 12, **dadurch gekennzeichnet,** daß als nachzuweisender Analyt oder als fixiertes Agens ein blutgruppenspezifischer Antikörper oder ein blutgruppenspezifisches Antigen verwendet wird.

14. Verfahren nach einem der Ansprüche 2 bis 13, **dadurch gekennzeichnet,** daß das bindende Agens auf einer die Sensoroberfläche überziehenden porösen Schicht fixiert wird, deren Porengröße nicht kleiner als die Größe der Teilchen der signalvermittelnden Substanz ist.

15. Verfahren nach einem der Ansprüche 1 bis 12, **dadurch gekennzeichnet,** daß die Bewegung der Partikel der partikulären Agenzien zur Sensoroberfläche durch Schwerkraft beschleunigt wird.

16. Verfahren nach Anspruch 15, **dadurch gekennzeichnet,** daß die Bewegung der Partikel der partikulären Agenzien durch Zentrifugation beschleunigt wird.

17. Verfahren nach einem der Ansprüche 1 bis 13 (14), **dadurch gekennzeichnet**, daß die Bewegung der Partikel der partikulären Agenzien oder der signalvermittelnden Substanzen zur Sensoroberfläche durch Kapillarkräfte beschleunigt wird.

18. Verfahren nach Anspruch 17, **dadurch gekennzeichnet,** daß die Kapillarkräfte durch eine poröse Sensoroberfläche hervorgerufen werden.

19. Verfahren nach einem der Ansprüche 1 bis 13, **dadurch gekennzeichnet,** daß partikuläre Agenzien mit magnetischen Eigenschaften verwendet werden und daß die Bewegung der Partikel der partikulären Agenzien zur Sensoroberfläche durch Anlegen eines magnetischen Feldes beschleunigt wird.

20. Verfahren nach einem der Ansprüche 1 bis 19, **dadurch gekennzeichnet,** daß als partikuläre Agenzien Zellen oder andere Partikel biologischer Herkunft verwendet werden.

21. Verfahren nach einem der Ansprüche 1 bis 20, **dadurch gekennzeichnet,** daß als Ausgangsstoffe für die partikulären Agenzien Latexpartikel oder andere künstliche hergestellte Partikel verwendet werden.

22. Verfahren nach einem der Ansprüche 1 bis 21, **dadurch gekennzeichnet,** daß die Sensoroberfläche als dünne Schicht auf einem planaren Substrat aufgebracht wird.

23. Verfahren nach einem der Ansprüche 1 bis 22, **dadurch gekennzeichnet,** daß die Sensoroberfläche oder/und die sie überziehende poröse Schicht nach erfolgter Messung regeneriert wird und für weitere Messungen verwendet werden kann.

24. Vorrichtung zur Durchführung des Verfahrens nach einem der Ansprüche 1 bis 23, mit einer Meßkammer (8), die einen Zulauf (1), eine mit einem Ablaufkanal (11) verbundene Öffnung (10) und eine in die Meßkammer (8) eingesetzte Hilfselektrode (2) aufweist, die an den Ausgang eines dritten Verstärkers (42) angeschlossen ist, während eine im Strömungsweg des Ablaufkanals (11) liegende Referenzelektrode (5) an den Eingang eines zweiten Verstärkers (52) angeschlossen ist, dessen Ausgang auf einen Eingang des dritten Verstärkers koppelt, und mit einem Sensor (3), der durch die Wand der Hilfselektrode (2) eingesetzt und an einen Eingang eines ersten Verstärkers (32) angeschlossen ist, dessen Ausgang einen Signalausgang der Vorrichtung bildet,
**dadurch gekennzeichnet,** daß die Sensoroberfläche oder die Sensoroberfläche und die sie überziehende poröse Schicht und das darauf immobilisierte bindende Agens auf einem zur einmaligen Verwendung gedachten Teil zusammengefaßt sind.

25. Vorrichtung nach Anspruch 24, **dadurch gekennzeichnet,** daß das zur einmaligen Verwendung gedachte Teil zusätzlich das signalvermittelnde Agenz oder die signalvermittelnde Substanz enthält.
